(19) Europäisches Patentamt / European Patent Office / Office européen des brevets

(11) **EP 1 911 736 B1**

(12) **EUROPÄISCHE PATENTSCHRIFT**

(45) Veröffentlichungstag und Bekanntmachung des Hinweises auf die Patenterteilung:
**20.10.2010 Patentblatt 2010/42**

(51) Int Cl.:
*C07C 41/06* (2006.01)    *C07C 43/04* (2006.01)
*G05D 11/13* (2006.01)

(21) Anmeldenummer: **07111946.5**

(22) Anmeldetag: **06.07.2007**

(54) **Verfahren zur Herstellung von Alkyl-tert.-butylethern mit optimierter Temperaturführung in den Reaktoren**

Method for manufacturing alkyl tert. butyl ethers with optimised temperature control in the reactors

Procédé pour la préparation d'ethers alkyls tertiobutyls par régulation de la température dans les réacteurs

(84) Benannte Vertragsstaaten:
**BE DE**

(30) Priorität: **19.07.2006 DE 102006033415**

(43) Veröffentlichungstag der Anmeldung:
**16.04.2008 Patentblatt 2008/16**

(73) Patentinhaber: **Evonik Oxeno GmbH**
**45772 Marl (DE)**

(72) Erfinder:
• **Santiago Fernandez, Silvia**
**33009 Oviedo (ES)**
• **Praefke, Jochen**
**45739 Oer-Erkenschwick (DE)**
• **Nierlich, Franz**
**45768 Marl (DE)**
• **Kaßler, Peter**
**44577 Castrop-Rauxel (DE)**
• **Rix, Armin**
**45770 Marl (DE)**

(56) Entgegenhaltungen:
EP-A- 0 729 085      DE-A1- 2 521 964
DE-A1- 10 334 001      US-A- 4 161 496

**Beschreibung**

Die Erfindung betrifft ein Verfahren zur Herstellung von Alkyl-tert.-Butylether (ATBE), insbesondere Methyl-tert.-butylether (MTBE) oder Ethyl-tert.-butylether (ETBE) aus technischen Mischungen von $C_4$-Kohlenwasserstoffen, die Isobuten und 1-Buten und gegebenenfalls weitere Verbindungen aufweisen, unter optimierten Temperaturbedingungen. Des Weiteren betrifft die Erfindung die Herstellung von 1-Buten mit einer Höchstkonzentration an 2-Butenen und Isobuten mittels des Verfahrens zur Herstellung von ATBE.

1-Buten, Isobuten, 2-Butene und deren Folgeprodukte werden in großen Mengen aus technischen $C_4$-Schnitten, beispielsweise dem $C_4$-Schnitt aus Steamcrackern oder FCC-Einheiten, gewonnen. Diese Gemische bestehen im Wesentlichen aus Butadien, den Monoolefinen Isobuten, 1-Buten und den beiden 2-Butenen sowie den gesättigten Kohlenwasserstoffen Isobutan und n-Butan. Wegen der geringen Siedepunktsunterschiede der Inhaltsstoffe und deren geringen Trennfaktoren ist eine destillative Aufarbeitung schwierig und nicht wirtschaftlich. Die Gewinnung von linearen Butenen und von anderen Produkten erfolgt daher meistens durch eine Kombination von chemischen Umsetzungen und physikalischen Trennoperationen.

Der erste Schritt, den dabei alle Aufarbeitungsvarianten gemeinsam haben, ist häufig die Entfernung des größten Teils des Butadiens. Kann Butadien gut vermarktet werden oder besteht ein Eigenverbrauch, wird es üblicherweise durch Extraktion oder Extraktivdestillation abgetrennt. Im anderen Fall wird es bis zu einer Restkonzentration von circa 2000 Massen-ppm (wppm) selektiv zu linearen Butenen hydriert. Zurück bleibt in beiden Fällen ein Kohlenwasserstoffgemisch (sogenanntes Raffinat I oder hydriertes Crack-$C_4$), das neben den gesättigten Kohlenwasserstoffen n-Butan und Isobutan die Olefine Isobuten, 1-Buten und 2-Butene (cis und trans) enthält.

Aus diesem Gemisch lässt sich durch Umsetzung des enthaltenen Isobutens mit Alkohol der entsprechende Alkyl-tert.-butylether (ATBE), insbesondere Ethyl-tert.-butylether (ETBE) und Methyl-tert.-butylether (MTBE) gewinnen. Nach der Umsetzung des Isobutens und Abtrennung des Alkyl-tert.-butylether verbleibt ein Kohlenwasserstoffgemisch (Raffinat II), das die linearen Butene und die gesättigten Kohlenwasserstoffe Isobutan und n-Butan enthält. Dieses Gemisch kann destillativ weiter aufgetrennt werden, beispielsweise in Isobutan und 1-Buten und ein Gemisch aus den beiden 2-Butenen und n-Butan. Aus der 1-Buten-haltigen Fraktion kann in weiteren Destillationsschritten 1-Buten in hoher Reinheit erhalten werden, welches nur noch geringe Mengen an Isobuten enthält. Dies ist notwendig, da 1-Buten im großen Maße als Comonomer in der Ethylenpolymerisation eingesetzt wird, wo Isobuten- und 2-Buten-Verunreinigungen unerwünscht sind. Typische Spezifikationen von 1-Buten beschränken daher den Gehalt an Isobuten im 1-Buten auf unter 2000 wppm. Verfahren zur Herstellung von ATBE aus Isobuten aufweisenden $C_4$-Kohlenwasserstofffraktionen weisen deshalb insbesondere dann eine hohe Wirtschaftlichkeit auf, wenn die Herstellung von ATBE ohne große Verluste an n-Butenen gelingt.

Für die Umsetzung von Isobuten mit Alkoholen, beispielsweise Methanol oder Ethanol, zu den entsprechenden tertiären Butylethern wurden diverse verfahrenstechnische Varianten entwickelt. Das technisch bedeutendste Verfahren ist die Umsetzung von Isobuten mit Methanol zu Methyltert.-butylether (MTBE), das hauptsächlich als Kraftstoffzusatz große Verwendung findet. Auf Grund der immer größeren Verfügbarkeit von Ethanol aus nachwachsenden Rohstoffen erhöht sich auch die Nachfrage nach ETBE als Kraftstoffzusatz. Die meisten technisch eingesetzten Verfahren zur Herstellung von ATBE sind zweistufige Verfahren, insbesondere solche, bei denen in der zweiten Stufe eine Reaktivdestillation eingesetzt wird.

In DE 25 21 964 wird ein zweistufiges Verfahren zur Herstellung von Alkyl-tert.-Butylethern beschrieben, bei dem in einer ersten Stufe Isobuten mit Alkohol umgesetzt wird, der entstandene Ether von Produktgemisch der ersten Stufe abgetrennt wird und der verbleibende Rest des Produktgemisches in eine zweite Reaktionsstufe geführt wird, in welcher verbleibendes Isobuten wiederum mit Alkohol umgesetzt wird.

US-A-4161496 beschreibt ein zwei-stufiges Verfahren zur Herstellung von Alkyl-tertiär-Butyl-Ethern.

EP-A-729085 beschreibt ein Verfahren zur Herstellung von Alkyl-tertiär-Butyl-Ethem, wobei lediglich die Konzentration der beiden Reaktanden mittels eines Kontrollsystem ermittelt wird und aufgrund dieser Daten eine optimale Mischung der beiden Reaktanden eingestellt wird.

In der noch nicht veröffentlichten Schrift DE 10 2005 062722 wird ein verbessertes Verfahren zur zweistufigen Herstellung von ETBE und 1-Buten beschrieben, bei dem zunächst eine Umsetzung von Teilen des in einer technischen Mischung enthaltenen Isobutens mit Ethanol in Gegenwart eines sauren Katalysators zu ETBE, dann eine Abtrennung der nicht umgesetzten $C_4$-Kohlenwasserstoffe aus dem Austrag der Umsetzung durch thermische Trennverfahren unter Erhalt einer ETBE aufweisenden Fraktion, dann eine destillative Trennung der $C_4$-Kohlenwasserstoffe in eine mindestens 1-Buten und Isobuten enthaltende Fraktion und eine nahezu Isobuten-freie, mindestens 2-Butene und n-Butan enthaltende Fraktion, und schließlich eine Umsetzung des in der 1-Buten und Isobuten enthaltenden Fraktion enthaltenen restlichen Isobutens mit Ethanol in Gegenwart eines sauren Katalysators zu ETBE, erfolgt.

Ein Nachteil des letztgenannten Verfahrens besteht darin, dass es zu Verlusten an 1-Buten durch Isomerisierung von 1-Buten zu 2-Butenen während der Umsetzung des Isobutens zum ETBE, insbesondere während der Umsetzung in der zweiten Stufe kommen kann. Dies hat zur Folge, dass 1-Buten erhalten wird, welches eine zu hohe Konzentration an 2-Butenen aufweist und welches somit nicht als Comonomer einsetzbar ist.

Es bestand daher die Aufgabe, ein verbessertes Verfahren zur Herstellung von ATBE bereitzustellen, mit welchem Verluste an 1-Buten durch Isomerisierung während der Umsetzung verhindert oder zumindest verringert werden können.

Überraschenderweise wurde gefunden, dass diese Aufgabe dadurch gelöst werden kann, dass die zweite Stufe der ATBE-Synthese in zumindest zwei Reaktoren durchgeführt wird, wobei die Umsetzung in Reaktoren bei Temperaturen und einer Zufuhr von Alkohol betrieben wird, die um maximal 10 % von den Werten abweichen, die aus den weiteren Verfahrensparametern ermittelt wurden.

Gegenstand der vorliegenden Erfindung ist deshalb ein Verfahren zur Herstellung von Alkyltert.-butylether aus einem Gemisch I, welches Isobuten und 1-Buten aufweist, durch sauer katalysierte Umsetzung des Isobutens mit Alkohol in zumindest zwei hintereinander geschalteten Reaktoren unter Erhalt eines Reaktionsproduktes II, welches gewünschte Konzentrationen an 2-Butenen und Isobuten nicht überschreitet, welches dadurch gekennzeichnet ist, dass die Umsetzung bei Temperaturen in °C am Eingang der Reaktoren und unter Zugabe einer Alkoholmenge zur Umsetzung durchgeführt wird, die jeweils um maximal 10 % von den Werten abweichen, die erhältlich sind durch die Schritte:

a) Ermitteln von mehreren Datensätzen bei denen Wertepaare für die Konzentrationen an 2-Butenen und Isobuten in Reaktionsprodukt II bestimmten Werten für die zugeführte Masse an Gemisch I zur Umsetzung, für die Isobuten-Konzentration in Gemisch I, für die Konzentration von 1-Buten im Gemisch I, für die der Umsetzung zugeführten Alkoholmenge und für die Katalysatorvolumina der Reaktoren zugeordnet sind, durch entsprechend häufiges Durchführen einer Umsetzung eines Gemischs I, wobei bei den Durchführungen die Konzentration von 1-Buten, die Isobuten-Konzentration, die Katalysatorvolumina, die zugeführte Masse an Gemisch I zur Umsetzung, die Temperaturen am Eingang der Reaktoren und/oder die der Umsetzung zugeführten Menge an Alkohol variiert wird, und
b) Auswahl eines Datensatzes, der Werte aufweist, die den tatsächlichen Bedingungen bei der Umsetzung in Bezug auf die zugeführte Masse an Gemisch I zur Umsetzung, die Isobuten-Konzentration in Gemisch I, die Konzentration von 1-Buten in Gemisch I und die Katalysatorvolumina mit einer Abweichung von maximal 5 % entsprechen und dem ein Wertepaar für die Konzentrationen an Isobuten und 2-Butenen im Reaktionsprodukt II zugeordnet ist, welche die gewünschten Konzentrationen an 2-Butenen und Isobuten in Bezug auf das enthaltene 1-Buten nicht überschreiten, wobei in dem Datensatz die Werte für die Temperaturen am Eingang der Reaktoren sowie für die der Umsetzung zuzuführenden Menge an Alkohol enthalten sind. Unter dem Katalysatorvolumen wird im erfindungsgemäßen Verfahren das Volumen des eingesetzten Katalysators in der Lieferform verstanden.

Mit dem erfindungsgemäßen Verfahren wird das Problem gelöst, dass die Umsetzung einerseits bis zu einem bestimmten Isobuten-Umsatz durchgeführt werden soll, andererseits die Umsetzung nicht mit zu langen Verweilzeiten durchgeführt werden soll, die zu einer erhöhten Bildung von 2-Butenen führen würde. Durch das erfindungsgemäße Verfahren können die optimalen Temperaturen in den Reaktoren auf einfache Weise in Abhängigkeit von den weiteren Verfahrensbedingungen eingestellt werden.

Der besondere Vorteil des erfindungsgemäßen Verfahrens besteht darin, dass die Umsetzung von Isobuten mit Alkohol in der zumindest zwei Reaktoren aufweisenden Umsetzung durch Einstellen der erhaltenen Temperaturen am Eingang der Reaktoren und der zuzugebenden Alkohol-Menge so durchgeführt werden kann, dass sowohl die gewünschte Grenzkonzentrationen von Isobuten als auch die gewünschte Grenzkonzentration von 2-Butenen im Reaktionsaustrag erreicht wird. Nach einer destillativen Abtrennung des ATBE kann ein 1-Buten aufweisender Strom erhalten werden, der den üblichen Spezifikationen für 1-Buten genügt. Ein solches 1-Buten kann z. B. als Comonomer bei der Herstellung von Polymeren eingesetzt werden.

Durch Einstellen der erhaltenen Temperaturen in den Reaktoren kann vermieden werden, dass während der Umsetzung des Isobutens 1-Buten zu 2-Butenen in einem Ausmaß isomerisiert, dass die gewünschte Konzentration an 2-Butenen überschritten, wobei gleichzeitig sichergestellt ist, dass die Isobutenkonzentration durch Umsetzung zum ATBE auf den gewünschten Wert reduziert wird. Durch Einstellen von Temperaturen, die durch die Zielfunktion erhalten werden, können insbesondere optimale Werte für Isobuten und 2-Butene innerhalb der Spezifikation erreicht werden.

Nachfolgend wird das erfindungsgemäße Verfahren beispielhaft beschrieben. Sind im nachfolgenden Text Bereiche bzw. Vorzugsbereiche angegeben, so sollen auch alle in diesen Bereichen liegenden, theoretisch möglichen Teilbereiche und Einzelwerte zum Offenbarungsgehalt der vorliegenden Erfindung gehören, ohne dass diese aus Gründen der besseren Übersichtlichkeit explizit genannt worden sind.

Das erfindungsgemäße Verfahren zur Herstellung von Alkyl-tert.-butylether aus einem Gemisch I, welches Isobuten und 1-Buten aufweist, durch sauer katalysierte Umsetzung des Isobutens mit Alkohol in zumindest zwei hintereinander geschalteten Reaktoren, vorzugsweise genau zwei Reaktoren unter Erhalt eines Reaktionsproduktes II, welches gewünschte Konzentrationen an 2-Butenen und Isobuten, vorzugsweise kleiner 5000 wppm an Isobuten und kleiner 1000 wppm an 2-Butenen, bevorzugt kleiner 2000 wppm an Isobuten und kleiner 500 wppm an 2-Butenen und besonders bevorzugt von 100 bis 1750 wppm an Isobuten und von 50 bis 400 wppm an 2-Butenen in Bezug auf das enthaltene 1-Buten nicht überschreitet, zeichnet sich dadurch aus, dass die Umsetzung bei Temperaturen in °C am Eingang der Reaktoren und unter Zugabe einer Alkoholmenge zur Umsetzung durchgeführt wird, die jeweils um maximal 10 %, vorzugsweise maximal 5 %, bevorzugt maximal 2 %, besonders bevorzugt maximal 0,5 % und ganz besonders bevorzugt gar nicht von den Werten abweichen, die erhältlich sind durch die Schritte:

a) Ermitteln von mehreren Datensätzen bei denen Wertepaare für die Konzentrationen an 2-Butenen und Isobuten in Reaktionsprodukt II bestimmten Werten für die zugeführte Masse an Gemisch I zur Umsetzung, für Isobuten-Konzentration in Gemisch I, für die Konzentration von 1-Buten im Gemisch I, für die der Umsetzung zugeführten Alkoholmenge und für die Katalysatorvolumina der Reaktoren zugeordnet sind, durch entsprechend häufiges Durchführen einer Umsetzung eines Gemischs I, wobei bei den Durchführungen die Konzentration von 1-Buten, die Isobuten-Konzentration, die Katalysatorvolumina, die zugeführte Masse an Gemisch I zur Umsetzung, die Temperaturen am Eingang der und/oder die der Umsetzung zugeführten Menge an Alkohol variiert wird, und

b) Auswahl eines Datensatzes, der Werte aufweist, die den tatsächlichen Bedingungen bei der Umsetzung in Bezug auf die zugeführte Masse an Gemisch I zur Umsetzung, die Isobuten-Konzentration in Gemisch I, die Konzentration von 1-Buten in Gemisch I und die Katalysatorvolumina mit einer Abweichung von maximal 5 %, vorzugsweise von maximal 2 %, bevorzugt von maximal 1 % und besonders bevorzugt ohne Abweichung entsprechen und dem ein Wertepaar für die Konzentrationen an Isobuten und 2-Butenen in Bezug auf das enthaltene 1-Buten im Reaktionsprodukt II zugeordnet ist, welche die gewünschten Konzentrationen an 2-Butenen und Isobuten in Bezug auf das enthaltene 1-Buten nicht überschreiten, wobei in dem Datensatz die genannten Werte für die Temperaturen am Eingang der sowie für die der Umsetzung zuzuführenden Menge an Alkohol enthalten sind. Das erfindungsgemäße Verfahren basiert also darauf, dass Werte für die Temperaturen und den Zulauf an Alkohol zur Umsetzung mit einer maximalen Abweichung von 10 % eingestellt werden, die durch Auswahl von Werten aus einer Vielzahl von Datensätzen, die durch Durchführung der Umsetzung mit verschiedenen Betriebsparametern ermittelt wurden, erhältlich sind bzw. auf diese Weise erhalten wurden.

Die Umsetzung des Isobutens wird als sauer katalysierte Reaktion durchgeführt. Als Alkohole können primäre, sekundäre, ein- oder mehrwertige Alkohole vorzugsweise mit 1 bis 5 C-Atomen, besonders bevorzugt Methanol oder Ethanol eingesetzt werden. Als Alkohol kann hochreiner Alkohol, reiner Alkohol oder Alkohol verwendet werden, der geringe Mengen an Verunreinigungen aufweist. Bevorzugt liegt die Reinheit des eingesetzten Alkohols, angegeben in Massen-% an Alkohol, über 90 %, besonders bevorzugt über 95 %, ganz besonders bevorzugt über 99 %. Der Gehalt an Wasser liegt bevorzugt unter 3 Massen-%, besonders bevorzugt unter 1 Massen-%, ganz besonders bevorzugt unter 0,3 Massen-%.

Die Umsetzung kann in der Gasphase oder in flüssiger Phase erfolgen. Vorzugsweise erfolgt die Umsetzung in flüssiger Phase.

Als Reaktoren, in denen der Alkohol mit dem Isobuten umgesetzt wird, können herkömmliche Festbettreaktoren (Rohrbündelreaktoren, adiabatische Festbettreaktoren, Kreislaufreaktoren) eingesetzt werden. Sie können mit oder ohne partielle Rückführung, wobei gegebenenfalls der Rückführungsstrom gekühlt werden kann, betrieben werden. Vorzugsweise wird keiner der Reaktoren mit einer Rückführung betrieben, da eine Rückführung zur stärkeren 2-Buten-Bildung durch Isomerisierung führen könnte.

Die Reaktoren können bei Temperaturen am Eingang der Reaktoren von 10 bis 160 °C, bevorzugt bei Temperaturen von 30 bis 60 °C, betrieben werden. Der Druck beträgt vorzugsweise 5 bis 50 bar$_{absolut}$ (bara), bevorzugt 10 bis 20 bara. Da das thermodynamische Gleichgewicht zwischen Alkohol/Isobuten und Ether bei tiefer Temperatur überwiegend auf der Seite des Ethers liegt, ist es bei Einsatz mehrerer Reaktoren bevorzugt, den ersten der Reaktoren bei höherer Temperatur (hohe Reaktionsgeschwindigkeit) als die Folgenden (Ausnutzung der Gleichgewichtslage) zu betreiben. Die Temperaturen werden so eingestellt, dass sie unter Berücksichtigung der maximalen Abweichungen den gemäß Schritt b) ermittelten Temperaturwerten entsprechen.

Das molare Verhältnis von Alkohol zu Isobuten im Zulauf zur Umsetzung liegt vorzugsweise im Bereich von 25 zu 1 bis 1 zu 1, besonders bevorzugt von 15 zu 1 bis 3 zu 1 und ganz besonders bevorzugt im Bereich von 10 zu 1 bis 5 zu 1. Auch hier wird der Wert für den Zulauf an Alkohol zur Umsetzung so eingestellt, dass er unter Berücksichtigung der oben genannten maximalen Abweichung dem gemäß Schritt b) ermittelten Wert entspricht.

Als Katalysator wird vorzugsweise ein fester Stoff, der weder im Einsatzstoffgemisch noch im Produktgemisch löslich ist, eingesetzt. Vorzugsweise weist der Katalysator saure Zentren an seiner Oberfläche auf. Der Katalysator sollte unter Reaktionsbedingungen keine sauren Stoffe an das Produktgemisch abgeben, weil dies zu Ausbeuteverluste führen kann.

Die Aktivität der Katalysatoren wird vorzugsweise so gewählt, dass sie unter Reaktionsbedingungen die Addition von Alkohol an Isobuten katalysieren, jedoch kaum die Addition an lineare Butene. Weiterhin sollten die Katalysatoren die Oligomerisierung von linearen Butenen und die Dialkyletherbildung aus zwei Molekülen eingesetzten Alkohols möglichst nicht oder nur wenig katalysieren. Im Hinblick auf eine hohe Ausbeute an 1-Buten sollte die Aktivität für die Isomerisierung von 1-Buten zu 2-Buten vorzugsweise gering sein.

Als feste Katalysatoren können beispielsweise Zeolithe, säureaktivierte Bentonite und/oder Tonerden, sulfonierte Zirkoniumoxide, Montmorillonite oder saure Ionenaustauscherharze verwendet werden.

Eine im erfindungsgemäßen Verfahren bevorzugte Gruppe von sauren Katalysatoren sind feste Ionenaustauscherharze, insbesondere solche mit Sulfonsäuregruppen. Geeignete Ionenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von Ionenaustauscherharzen mit Sulfonsäuregruppen verwendet. Die Harze können gelförmig, makroporös oder schwammförmig hergestellt werden.

Die Eigenschaften dieser Harze, insbesondere spezifische Oberfläche, Porosität, Stabilität, Quellung bzw. Schrumpfung und Austauschkapazität, können durch den Herstellprozess variiert werden.

Bei Einsatz von Ionenaustauscherharzen als Katalysatoren können die Ionenaustauscherharze im erfindungsgemäßen Verfahren in ihrer H-Form eingesetzt werden. Stark saure Harze des Styrol-Divinylbenzol-Typs werden u. a. unter folgenden Handelsnamen verkauft: Duolite C20, Duolite C26, Amberlyst 15, Amberlyst 35, Amberlite IR-120, Amberlite 200, Dowex 50, Lewatit SPC 118, Lewatit SPC 108, K2611, K2621, OC 1501.

Das Porenvolumen beträgt vorzugsweise von 0,3 bis 0,9 ml/g, insbesondere von 0,5 bis 0,9 ml/g. Die Korngröße des Harzes beträgt bevorzugt von 0,3 mm bis 1,5 mm, insbesondere von 0,5 mm bis 1,0 mm. Die Korngrößenverteilung kann enger oder weiter gewählt werden. So können beispielsweise Ionenaustauscherharze mit sehr einheitlicher Korngröße (monodisperse Harze) eingesetzt werden. Die Kapazität des Ionenaustauscher beträgt, bezogen auf die Lieferform, bevorzugt von 0,7 bis 2,0 eq/1, insbesondere von 1,1 bis 2,0 eq/l bzw. vorzugsweise von 0,5 bis 5,5 mol/kg, insbesondere von 0,8 bis 5,5 mol/kg (Die Angaben zur Kapazität in mol/kg beziehen sich auf das jeweils bis zur Gewichtskonstanz im warmen Stickstoffstrom bei z. B. 105 °C getrocknete Ionentauscherharz.).

Es kann vorteilhaft sein, wenn das bei der Umsetzung erhaltene Reaktionsprodukt destillativ in einen Alkyltert.-butylether III und einen 1-Buten aufweisenden Strom IV getrennt wird. Die Trennung kann z. B. dadurch erfolgen, dass das Reaktionsprodukt II in eine Destillationskolonne K1 eingespeist wird. Die Kolonne kann mit einem Sumpfverdampfer und einem Kondensator für das Kopfprodukt ausgerüstet sein. Als Sumpfprodukt der Destillationskolonne wird ATBE und gegebenenfalls überschüssiger Alkohol erhalten. Als Kopfprodukt wird ein 1-Buten aufweisender Strom IV erhalten, welches teilweise als Rücklauf in die Kolonne zurückgefahren werden kann. Der andere Teil des Stroms IV kann direkt verwendet werden oder einer weiteren Aufreinigung zugeführt werden.

Die Kolonne K1 weist vorzugsweise mehr als 20, bevorzugt mehr als 25, besonders bevorzugt mehr als 30 theoretischen Trennstufen auf. Das Rücklaufverhältnis ist, in Abhängigkeit von der realisierten Stufenzahl, vorzugsweise kleiner-gleich 2, besonders bevorzugt kleiner 1. Die Kondensation kann gegen Kühlwasser oder Luft durchgeführt werden. Zur Beheizung des Verdampfers der Kolonne kann z. B. Dampf eingesetzt werden. Es kann vorteilhaft sein, wenn der Zulaufstrom zur Kolonne zumindest teilweise vorverdampft in die Kolonne zu leiten oder direkt in die Kolonne zu flashen. Bevorzugt wird dafür dem Zulaufstrom in einem externen Wärmeübertrager Wärme, z. B. durch Nutzung von Abwärme, zugeführt. Zum Erzielen einer teilweisen Verdampfung ist ein Kettle-Verdampfer die bevorzugte Ausführungsform des Wärmeübertragers. Es kann außerdem vorteilhaft sein, wenn ein mit Prozess- oder Abwärme auf niedrigerem Temperaturniveau beheizter Zwischenverdampfer im unteren Teil der Kolonne eingesetzt wird.

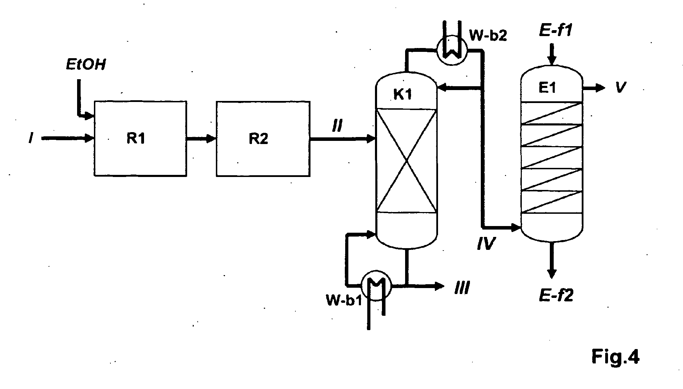

Weist das Kopfprodukt IV der Kolonne K1 noch Restmengen an Alkohol auf, so kann es vorteilhaft sein, diese in mindestens einem zusätzlichen Verfahrensschritt zu entfernen. Verfahren zur Abtrennung von Alkoholen, insbesondere von Methanol oder Ethanol, gehören zum Stand der Technik. Die Abtrennung kann beispielsweise über Adsorption an Molsieben, Membranverfahren, Schleppmitteldestillation oder Extraktion erfolgen. In Fig. 4 ist eine Apparatur zur Durchführung des erfindungsgemäßen Verfahrens dargestellt, bei dem die Abtrennung des Alkohols mittels Extraktion erfolgt. Besonders elegant kann die Abtrennung in einem Extraktionsschritt durch Extraktion des Alkohols mit Wasser erfolgen. Diese Wäsche erfolgt nach den bekannten Standardverfahren der Technik, beispielsweise in einer Extraktionskolonne oder in einer Kaskade von Mixern und Trennbehältern. Gegenüber den anderen Verfahren weist sie verschiedene Vorteile auf, beispielsweise geringes Investment und niedrige Betriebskosten.

Bei Einsatz von Methanol oder Ethanol als Alkohol werden Restmengen des Alkohols aus dem Strom IV bevorzugt in einer Extraktionskolonne mit Wasser entfernt. Der Restgehalt an Alkohol im Strom IV wird dabei bevorzugt auf unter 0,2 %, besonders bevorzugt auf unter 500 wppm, ganz besonders bevorzugt auf unter 50 wppm abgesenkt. Eine geeignete Extraktionskolonne weist bevorzugt 25 bis 2, besonders bevorzugt 15 bis 5 theoretische Trennstufen auf und wird bevorzugt bei Temperaturen von 10 bis 90 °C und Drücken von mindestens einem bar über dem Dampfdruck der im Strom IV enthaltenen $C_4$-Kohlenwasserstoffe, insbesondere 1-Buten.

Das mit Alkohol beladene Waschwasser aus der Extraktion kann bevorzugt in einer separaten Einheit aufgearbeitet und zumindest teilweise in die Extraktion zurückgeführt werden. Die Aufarbeitung kann beispielsweise durch eine Destillation erfolgen, bei der eine praktisch alkoholfreie Wasserfraktion im Sumpf und eine alkoholreiche Fraktion als Kopfprodukt erhalten wird.

Bevorzugt wird das Kopfprodukt IV der Destillationskolonne K1 in eine Extraktionskolonne überführt, in die im Gegenstrom ein Extraktionsmittel, wie z. B. Wasser, über einen am Kopf befindlichen Zulauf eingespeist wird. Das Extraktionsmittel kann über den Ablauf am Sumpf der Kolonne entnommen werden. Am Kopf der Kolonne wird als Produkt der Extraktion ein Strom V erhalten, der vorzugsweise die oben genannten Anteile an Alkohol aufweist.

Durch das erfindungsgemäße Verfahren können 1-Buten aufweisende Ströme IV oder V hergestellt werden, die jeweils bezogen auf das 1-Buten weniger als 5000 wppm Isobuten und weniger als 1000 wppm 2-Butene, vorzugsweise weniger als 2000 wppm Isobuten und weniger als 500 wppm 2-Butene und besonders bevorzugt von 100 bis 1750 wppm an Isobuten und von 50 bis 400 wppm an 2-Butenen aufweisen.

Weist das Gemisch I neben Isobuten und 1-Buten weitere Inhaltsstoffe, wie z. B. Isobutan und gegebenenfalls Leichtsieder auf, so kann es vorteilhaft sein den Strom V in einer weiteren Destillationskolonne K2, in der als Sumpfprodukt sehr reines 1-Buten und als Kopfprodukt eine isobutanreiche Fraktion erhalten wird, die zudem Leichtsieder (beispielsweise $C_3$-Kohlenwasserstoffe) enthalten kann, aufzutrennen.

Bevorzugt wird die Trennung in einer Superfraktionierkolonne als Destillationskolonne K2 durchgeführt. Der Zulauf zu dieser Kolonne erfolgt vorzugsweise in die obere Hälfte, bevorzugt in die untere Hälfte der oberen Hälfte der Kolonne K2. Wegen der engen Siedelage des zu trennenden Gemisches wird die Kolonne mit vorzugsweise mehr als 100, bevorzugt mehr als 125, besonders bevorzugt mehr als 150 und ganz besonders bevorzugt von 150 bis 200 theoretischen Trennstufen durchgeführt. Das Rücklaufverhältnis (Rücklaufmenge zu Destillatabnahme) ist, in Abhängigkeit von der realisierten Stufenzahl und vom Betriebsdruck, vorzugsweise kleiner-gleich 100, bevorzugt kleiner 70, besonders bevorzugt kleiner 60. Ganz besonders bevorzugt beträgt das Rücklaufverhältnis von 30 bis 60. Die Kondensation kann gegen Kühlwasser oder Luft durchgeführt werden. Der Destillatbehälter wird vorzugsweise als flüssig-flüssig-Abscheider ausgeführt. Dadurch kann gegebenenfalls im Zulaufstrom enthaltenes Wasser als zweite Phase im Destillatbehälter abgetrennt werden und es kann ein technisch wasserfreies Sumpfprodukt erhalten werden.

Zur Beheizung des Verdampfers der Kolonne K2 kann ein üblicher Wärmeträger, wie z. B. Dampf oder Warmwasser sowie bevorzugt Abwärme aus anderen Prozessen eingesetzt werden. In letzterem Fall kann es vorteilhaft sein, die Kolonne mit mehr als einem Verdampfer auszustatten. Die Kolonne K2 wird bevorzugt als einfache Kolonne mit mindestens einem Verdampfer und mindestens einem Kondensator ausgerüstet. Wegen des hohen Energiebedarfs und der kleinen Temperaturdifferenz zwischen Sumpf und Kopf der Kolonne sind Energiesparschaltungen besonders bevorzugte Ausführungsformen. Exemplarisch sei hier auf die Methode der Brüdenverdichtung verwiesen. Eine weitere besonders bevorzugte Schaltung ist die Zweidruckschaltung (double effect distillation) in Integration mit einer zweiten Kolonne. Die zweite Kolonne kann bevorzugt eine parallelgeschaltete Kolonne mit gleicher oder unterschiedlicher Trennaufgabe sein. Dabei wird eine der Kolonnen bei so hohem Druck gefahren, dass ihre Kondensationstemperatur zur Beheizung der anderen Kolonne ausreicht. Bei der wärmetechnischen Verschaltung von Kolonnen mit unterschiedlichen Trennaufgaben kann prinzipiell jede geeignete Kolonne aus dem erfindungsgemäßen Verfahren aber auch eine Kolonnen, die außerhalb des erfindungsgemäßen Verfahren am Anlagenstandort vorhanden ist, mit der Kolonne K2 verschaltet werden.

In einer weiteren bevorzugten Ausführungsform des erfindungsgemäßen Verfahrens werden in einer ersten Destillationskolonne K2 Leichtsieder als Kopfprodukt abgetrennt; im Sumpf der Kolonne wird eine Mischung erhalten, die hauptsächlich 1-Buten und Isobutan enthält. In einer zweiten Kolonne K3 wird diese Sumpfmischung in 1-Buten, welches als Sumpfprodukt anfällt, und eine Isobutan-reiche Fraktion (Kopfprodukt), getrennt.

So hergestelltes, besonders reines 1-Buten enthält vorzugsweise weniger als 5000 wppm (Massen-ppm), bevorzugt weniger als 2000 wppm und besonders bevorzugt weniger als 1500 wppm Isobuten und ist ein gefragtes Zwischenprodukt. Es wird beispielsweise als Comonomer bei der Herstellung von Polyethylen (LLDPE oder HDPE) sowie von Ethylen-Propylen-Mischpolymeren eingesetzt. Es findet weiterhin Einsatz als Alkylierungsmittel und ist Ausgangsstoff für die Herstellung von Butan-2-ol, Butenoxid, Valeraldehyd. Eine weitere Verwendung des erfindungsgemäß hergestellten nahezu isobutenfreien 1-Butens ist die Herstellung von n-Buten-Oligomeren, insbesondere nach dem Octol-Prozess.

Neben dem 1-Buten fallen üblicherweise (je nach Ausgangszusammensetzung der $C_4$-Kohlenwasserstoffe) isobutanreiche Fraktionen an. Diese können weiter, vorzugsweise zu reinem Isobutan, aufgereinigt werden. Das bei der Aufarbeitung gewonnene Isobutan hat vorzugsweise eine Reinheit von mindestens 90 Massen-% Isobutan, besonders bevorzugt 95 Massen-% Isobutan und enthält bevorzugt weniger als 1000 wppm, besonders bevorzugt weniger als 200 wppm Olefine. Eine Aufreinigung zu reinem Isobutan kann beispielsweise durch vollständige Hydrierung der noch enthaltenen Alkene zu Alkanen und anschließende Destillation erfolgen.

Der in Kolonne K1 als Sumpfprodukt anfallende Alkyl-tert.-butylether, der gegebenenfalls noch Restmengen an Alkohol enthält, kann für verschiedene Zwecke genutzt werden. Neben dem Einsatz als Komponente für Ottokraftstoffe findet er beispielsweise Verwendung als Lösungsmittel. Durch Rückspaltung der Alkyl-tert.-Butylether ist Isobuten hoher Reinheit erhältlich.

Der bei Verwendung von Methanol als Alkohol anfallendes MTBE kann beispielsweise, neben der Verwendung als Komponente im Ottokraftstoff, als Lösungsmittel genutzt werden. Zur Gewinnung von MTBE hoher Reinheit, welcher bevorzugt als Lösungsmittel eingesetzt wird, kann der im Prozess anfallende MTBE destillativ weiter aufgereinigt werden. Dabei wird der Gehalt an gegebenenfalls in geringer Menge enthaltenen Verunreinigungen (beispielsweise Methyl-sec.-butylether, $C_8$-Kohlenwasserstoffe, tert.-Butanol, Alkohole) reduziert.

Die Rückspaltung von MTBE zur Gewinnung von Isobuten wird beispielsweise in DE 100 20 943 beschrieben. Die dabei erhaltene Reinheit des Isobutens ist unter anderem abhängig vom Anteil an Methyl-sec.-butylether im MTBE. Je nach Anforderungen wird daher ein unterschiedlich intensiv vorgereinigtes MTBE für die Rückspaltung eingesetzt. Es kann vorteilhaft sein, wenn in Schritt a) zumindest 10, vorzugsweise von 100 bis 1000000, bevorzugt 500 bis 100000, besonders bevorzugt 1000 bis 20000, besonders bevorzugt 5000 bis 10000 Datensätze. Ganz besonders bevorzugt werden in Schritt a) zumindest 6000, vorzugsweise 6000 bis 10000 Datensätze ermittelt.

Die Anzahl der zu ermittelnden Datensätze ist von der Größe der Bereiche, in denen die Parameter variieren können sollen, und von der Anzahl der Werte in diesen Bereichen abhängig. Je größer die Anzahl der Werte in den Bereichen desto mehr mögliche Betriebsparameter werden durch die größere Anzahl von ermittelten Datensätzen zur Verfügung gestellt bzw. abgedeckt.

Es kann vorteilhaft sein, wenn bei der Ermittlung der Datensätze nicht alle Parameter variiert werden. Dies kann insbesondere dann vorteilhaft sein, wenn bestimmte Parameter bei Betrieb des Verfahrens feststehen oder für einen längeren Zeitraum, wie z. B. mehrere Wochen, Monate oder Jahre festgelegt werden. Soll das erfindungsgemäße Verfahren in einer bestehenden Anlage eingesetzt werden, so kann es z. B. vorteilhaft sein, die Katalysatorvolumina festzulegen, da, zumindest bei Einsatz von Festbettreaktoren, die Katalysatorvolumina über einen langen Zeitraum im Wesentlichen konstant bleiben. Wird das erfindungsgemäße Verfahren mit einem Gemisch I durchgeführt, bei dem die Konzentration von 1-Buten über einen längeren Zeitraum im Wesentlichen konstant ist, kann es vorteilhaft sein, diesen Parameter nicht zu variieren. Wenn sowohl die Katalysatorvolumina als auch die Konzentration an 1-Buten über einen längeren Zeitraum im Wesentlichen konstant bleiben, kann es besonders vorteilhaft sein, sowohl die Katalysatorvolumina als auch die Konzentration von 1-Buten bei der Ermittlung der Datensätze nicht zu variieren. Ebenso festgelegt werden können weitere Parameter, wie z. B. die Isobuten-Konzentration, wenn diese über einen längeren Zeitraum konstant ist. Auf diese Weise kann die Anzahl der zu ermittelnden Datensätze deutlich reduziert werden oder bei gleicher Anzahl der Datensätze die Bereiche oder die Anzahl der Werte in den Bereichen vergrößert werden.

Das Ermitteln der Datensätze durch Durchführen einer Umsetzung eines Gemisches I kann durch Ausführen der entsprechenden Umsetzung oder durch Simulation der entsprechenden Umsetzung erfolgen. Vorzugsweise erfolgt die Ermittlung der Datensätze durch Simulation einer entsprechenden Anzahl von Umsetzungen. Insbesondere bei der Ermittlung von über 100 Datensätzen erfolgt die Ermittlung durch Simulation. Bei 100 oder weniger zu ermittelnden Datensätzen kann es auch vorteilhaft sein, die Datensätze durch Ausführung der Umsetzung und Analyse der erhaltenen Produkte zu ermitteln.

Die Simulation kann z. B. durch Verwendung von Computerprogrammen, wie z. B. Aspen Plus (Version 12.1) auf bekannte Weise erfolgen. Zur Simulation können Stoffdaten, wie z. B. Reaktionsenthalpie, Dichte, Wärmekapazität etc. aus Stoffdatenbanken, wie z. B. Detherm, oder solche, die experimentell ermittelt wurden, eingesetzt werden. Für die Simulation können alle möglichen oder nur die wesentlichen Reaktionen betrachtet werden. Vorzugsweise werden bei der Simulation alle wesentlichen Reaktionen und Nebenreaktionen, die bei der Umsetzung ablaufen können, berücksichtigt. Bei der Umsetzung von Isobuten mit Alkohol zu ATBE kommen für die Simulation vorzugsweise die nachfolgend beispielhaft für die Umsetzung von Isobuten mit Ethanol aufgeführten, schematischen Reaktionen in betracht.

$$\text{ETBE-Synthese:} \qquad \text{Isobuten + Ethanol} \underset{r1'}{\overset{r1}{\rightleftarrows}} \text{ETBE}$$

$$\text{Isobuten-Dimerisierung:} \qquad \text{Isobuten + Isobuten} \underset{r2'}{\overset{r2}{\rightleftarrows}} \text{Diisobuten}$$

$$\text{Diethylether-Bildung:} \qquad \text{Ethanol + Ethanol} \underset{r3'}{\overset{r3}{\rightleftarrows}} \text{Diethylether + Wasser}$$

$$\text{Tert.-Butanol-Synthese:} \qquad \text{Isobuten + Wasser} \underset{r4'}{\overset{r4}{\rightleftarrows}} \text{tert.-Butanol}$$

ESBE-Synthese:    2-Buten + Ethanol $\underset{r5'}{\overset{r5}{\rightleftharpoons}}$ ESBE

1-Buten-Isomerisierung:    1-Buten $\underset{r6'}{\overset{r6}{\rightleftharpoons}}$ 2-Buten

In diesem Reaktionsschema steht ESBE für Ethyl-sec.-butylether. Bei Einsatz von anderen Alkoholen als Ethanol müssten die entsprechenden Reaktionen berücksichtigt werden. So würde beim Einsatz von Methanol als Alkohol in der ersten Reaktion Methyl-tert.-butylether, in der dritten Reaktion Dimethylether und in der fünften Reaktion Methyl-sec.-butylether entstehen. Zur Vereinfachung können bei der ATBE Synthese insbesondere die ATBE-Synthese, also ETBE- bzw. MTBE-Synthese und die 1-Buten-Isomerisierung berücksichtigt werden.

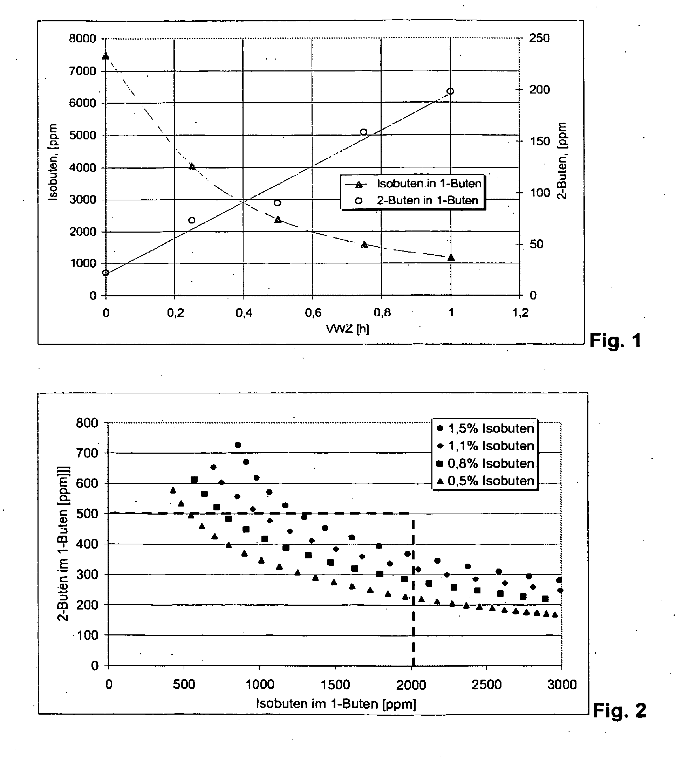

Die in der sechsten Reaktion durch Isomerisierung gebildeten 2-Butene verbleiben im 1-Buten, was zu Verlusten an 1-Buten und einer Gefährdung der 1-Buten-Spezifikation führt. In Fig. 1 ist ein Konzentrationsprofil für eine Umsetzung von Isobuten mit Ethanol zu ETBE bei 42 °C dargestellt. Das eingesetzte Gemisch aus 1-Buten und Isobutan (molares Verhältnis 9 zu 1) wies einen Anteil an Isobuten von 0,75 Massen-% auf.

Vorzugsweise werden für die oben genannten Reaktionen die Kinetiken ermittelt. Dies kann auf eine dem Fachmann bekannt Weise erfolgen. Vorzugsweise werden bei der Ermittlung der Kinetiken die Aktivitäten z. B. unter Verwendung eines Aktivitätskoeffizientenmodells, wie z. B. NRTL-RK (einem in der Simulationssoftware Aspen Plus vorhandenen Grundmodell) berechnet. Auf diese Weise können für die im Reaktionssystem wesentlichen Reaktionen die Reaktionsgeschwindigkeiten r1 bis r6' ermittelt werden.

Für die Geschwindigkeitskonstanten kann das Arrhenius-Gesetz

$$k_i = k_{0,i} \cdot \exp\left(\frac{-E_{A,i}}{R \cdot T}\right)$$

angenommen werden. T ist dabei die Temperatur in [K]. Durch Verwendung dieser Formeln können dann angepasste kinetische Parameter erhalten werden.

Die angepassten kinetischen Parameter können dann für die Simulation der Konzentrationen am Ausgang der Reaktoren ausgehend von den Eingangskonzentrationen und den Bedingungen in den technischen Reaktoren eingesetzt werden.

Wird bei der Simulation das Katalysatorvolumen in den beiden Reaktoren als fest mit einem bestimmten Wert angenommen, so können die zwei technischen Reaktoren mit der bekannten Differentialgleichung für die Bilanz eines polytropen Rohrreaktors berechnet werden. Für die Wärmebilanz können die Enthalpien der jeweiligen Reaktionen verwendet werden.

Unter Verwendung der ermittelten Kinetiken können nun verschiedene Betriebszustände simuliert werden, wobei dabei jeweils einer oder mehrere Parameter variiert werden. So kann beispielsweise die Eingangstemperatur am ersten Reaktor konstant gehalten werden und die Eingangstemperatur am zweiten Reaktor variiert werden. Aus der Simulation der verschiedenen Betriebszustände werden dann jeweils Datensätze erhalten, denen ein Wertepaar für die Konzentrationen an 2-Butenen und Isobuten im Reaktionsprodukt II zugeordnet ist.

Unabhängig davon, ob die Datensätze durch Simulation oder Ausführung einer entsprechenden Anzahl von Umsetzungen erhalten wurden, erfolgt in Schritt b) eine Auswahl aus den ermittelten Datensätzen von solchen Datensätzen, die in Bezug auf die zugeführte Masse an Gemisch I, Isobutenkonzentration in Gemisch I, 1-Buten-Konzentration in Gemisch I und die Katalysatorvolumina mit einer Abweichung von maximal 5 % den tatsächlichen Bedingungen entsprechen und welche die gewünschten Werte für die Konzentrationen an 2-Butenen und Isobuten aufweisen. Entsprechen mehrere Datensätzen diesen Kriterien kann eine Gewichtung in Bezug auf die Konzentrationen an 2-Butenen und 1-Butenen vorgenommen werden. Wichtig ist, dass durch ein Ausführung der Umsetzung bei Temperaturen und einer Alkoholzugabe, die den im Datensatz erhaltenen Werten für diese Parameter entsprechen, ein Reaktionsprodukt erhalten wird, welches die gewünschten Konzentrationen nicht überschreitet.

Die Auswahl des Datensatzes kann von Hand oder mit Hilfe eines Computerprogramms erfolgen. Vorzugsweise erfolgt die Auswahl unter Verwendung einer Zielfunktion. Diese kann z. B. so ausgelegt sein, dass sie im spezifikationsgerechten Bereich streng monoton von der optimierenden Größe abhängt und nahe der Spezifikationsgrenze exponentiell ansteigt und den optimalen Punkt in sichere Bereiche drängt. Eine solche Funktion kann z. B. die folgende

Form haben:

$$Z_{(y)} = \exp[\text{Faktor} \cdot (\text{aktuelle Konzentration}_{(y)} - \text{Spezifikationskonzentration}_{(y)})] \qquad \text{(I)}$$

mit y = betrachtete Komponente.

Da verschiedene Konzentrationen in das Optimierproblem der Zielfunktion eingehen können, kann es vorteilhaft sein, wenn die einzelnen Komponentenbeiträge in der Zielfunktion vorher vergleichbar gemacht werden. Die Spezifikationsgrenzen und Konzentrationen der jeweiligen Substanz werden dazu vorzugsweise auf ihren Mittelwert im Intervall aller möglichen Betriebspunkte bezogen (normiert). Dann werden diese normierten Konzentrationen im Verhältnis zur Breite des Intervalls aller möglichen Betriebspunkte vergleichbar gemacht. Die schmalen Intervalle werden gespreizt, so dass sie ein ähnliches Werteband abdecken, wie die breiteren. Die Komponentenbeiträge zur Zielfunktion werden durch eine Exponentialfunktion vorzugsweise so berechnet, dass wegen der spezifischen Spreizung das Argument der Exponentialfunktion an der Spezifikationsgrenze Null ist, unter der Grenze negativ. Diese Komponentenbeiträge werden zur Gesamt - Zielfunktion multipliziert.

Die Faktoren für 2-Butene und Isobuten lassen sich dabei wie nachfolgend beispielhaft beschrieben ermitteln:

Die Stellgrößen (Eingangsvariablen) des Optimierproblems können z. B. die beiden Temperaturen im Reaktoreingang sein. In Modellrechnungen können für die zulässigen Intervalle dieser beiden Eingangsvariablen die relativen 2-Buten- und Isobuten-Konzentrationen (bezogen auf 1-Buten) als Ergebnisse ermittelt werden (für die 2-Butene wird nachfolgend der Index 2B und für das Isobuten der Index iB verwendet. Aus den Ergebnissen kann der Mittelwert und die Höchst - und Tiefstwerte für die Konzentrationen ermittelt werden.

Für die Reaktoreingangstemperaturen des ersten Reaktors T1 und des ersten Reaktors T2 können die zulässigen Intervalle z. B. $T_1 \in [30:50]\,°C$ und $T_2 \in [30:70]\,°C$ betragen. Aus den Berechnungen für diese Temperaturintervalle können die Höchstwerte (Max), die Tiefstwerte (Min) und die Mittelwerte (Mittel) erhalten werden. Diese sowie die gewünschte Grenzkonzentration (Grenze) sind für die 2-Butene und das Isobuten nachfolgend in Massen-ppm (wppm), jeweils bezogen auf 1-Buten, angegeben.

$Min_{2B}$: 283 wppm, $Max_{2B}$: 3729 wppm, $Mittel_{2B}$: 1363 ppm, $Grenze_{2B}$: 500 ppm
$Min_{iB}$: 527 wppm, $Max_{iB}$: 5423 wppm, $Mittel_{iB}$: 2384 ppm, $Grenze_{iB}$: 2000 ppm

Aus diesen Werten kann die Spreizung für die 2-Butene ($Spreizung_{2B}$) berechnet werden, wobei für die Spreizung von Isobuten ($Spreizung_{iB}$) ein Wert von 10 festgesetzt wurde. Es wurden folgende Werte erhalten:

$$Spreizung_{2B} = Grenze_{iB} / Grenze_{2B} * (Max_{iB} - Min_{iB}) / Max_{2B} - Min_{2B}) = 5{,}69$$

$$Spreizung_{iB} = 10$$

Vorteilhafterweise wird jedes Konzentrationsergebnis durch Bezug auf den Mittelwert vergleichbar gemacht (normiert). Auch die Spezifikationsgrenzen werden durch den Mittelwert geteilt. Da die Intervalle der beiden Ergebnisse unterschiedlich breit sind, werden sie auf ein einheitliches Maß gespreizt. Die normierten Werte sind jeweils durch ein vorangestelltes N kenntlich gemacht. Es werden bei der vorliegenden beispielhaften Berechnung die folgenden Werte erhalten:

$NMin_{2B}$: 21 %, $NMax_{2B}$: 273 %, $NGrenze_{2B}$: 37%
$NMin_{iB}$: 38 %, $NMax_{iB}$: 392 %, $NGrenze_{iB}$: 144%

Unter Berücksichtigung der normierten Werte und durch Ersatz des Faktors durch die Spreizung wird aus Formel I die nachfolgende Formel Ia für die Zielfunktion erhalten.

$$Z_y = \exp[Spreizung_y (X_y/Mittel_y - Grenze_y/Mittel_y)] \qquad \text{(Ia)}$$

mit X = Konzentration der Komponente y bezogen auf 1-Buten und y $\in$ [2B, iB] und

**[0066]** Die Gesamt-Zielfunktion (II) setzt sich multiplikativ aus den Komponentenbeiträgen zusammen, die so geformt sind, dass sie im spezifikationsgerechten Intervall streng monoton von den normierten Konzentrationen abhängen, nahe der Grenze aber exponentiell ansteigen. Die kombinierte Gesamt-Zielfunktion Z für Isobuten und 2-Butene beträgt damit:

$$Z = (1 + Z_{(iB)})(1 + Z_{(2B)}) \qquad\qquad\qquad \textbf{(II)}$$

Als sicherer Grenzewert, bei der die Spezifikationsgrenzen (z. B. 2000 wppm Isobuten und 500 wppm für 2-Butene) eingehalten werden, wurde ein Wert für Z < 2,07 festgelegt. Der Datensatz, der den niedrigsten Wert für Z aufweist, kann als optimaler Datensatz angesehen werden. Die Temperaturwerte und die zuzugebende Alkoholmenge dieses optimalen Datensatzes werden ganz besonders bevorzugt im erfindungsgemäßen Verfahren eingesetzt.

Die Auswahl des Datensatzes unter Verwendung einer Zielfunktion erfolgt besonders bevorzugt mit Hilfe eines Computerprogramms, in dem die Zielfunktion hinterlegt ist.

**Einsatzstoffe**

Als Gemisch I, welches in dem erfindungsgemäßen Verfahren eingesetzt werden kann, können alle üblicherweise zur Verfügung stehenden technischen $C_4$-Kohlenwasserstoffgemische eingesetzt werden. Geeignete isobutenhaltige $C_4$-Ströme sind beispielsweise Leichtbenzinfraktionen aus Raffinerien, $C_4$-Fraktionen aus Crackern (beispielsweise Steamcracker, Hydrocracker, Katcracker), Gemische aus Fischer-Tropsch-Synthesen, Gemische aus der Dehydrierung von Butanen, Gemische aus Skelettisomerisierung linearer Butene und Gemische, entstanden durch Metathese von Olefinen. Diese Techniken sind in der Fachliteratur beschrieben. (K. Weissermel, H. J. Arpe, Industrielle Organische Chemie, Wiley-VCH, 5. Auflage, 1998, Seiten 23 bis 24; 65 bis 99; 122 bis 124). Vorzugsweise werden in dem erfindungsgemäßen Verfahren solche $C_4$-Kohlenwasserstoffgemische als Gemische I eingesetzt, die bei der Abtrennung der nicht umgesetzten C4-Kohlenwasserstoffe aus dem Reaktionsgemisch einer ersten Stufe eines ATBE-Verfahrens erhalten werden.

Bevorzugt eingesetzte Gemische I weisen einen nur sehr geringen Anteil an 2-Butenen auf. So werden vorzugsweise solche Gemische I eingesetzt, die von 0 bis kleiner 500 wppm, bevorzugt 0 bis kleiner 100 wppm, besonders bevorzugt von 0 bis 50 wppm an 2-Butenen in Bezug auf die enthaltenen 1-Butene aufweist. Ganz besonders bevorzugt werden solche Gemische I eingesetzt, die keine 2-Butene (also keine bzw. unterhalb der Nachweisgrenze) aufweisen. Sollen in dem erfindungsgemäßen Verfahren technische Mischungen von $C_4$-Kohlenwasserstoffen, insbesondere solche wie nachfolgend beschrieben eingesetzt werden, so weist das erfindungsgemäße Verfahren vorzugsweise zumindest ein Vorverfahren auf, mit dem die Konzentration an 2-Butenen in Bezug auf das 1-Buten in der technischen Mischung auf eine der oben genannten Konzentrationen reduziert wird. Solche Vorverfahren können einfache thermische Trennverfahren, wie z. B. eine Destillation sein. Ein solches Vorverfahren kann auch Teil eines Gesamtverfahrens sein, wenn ein Produktstrom dieses Gesamtverfahrens ein Gemisch I ist, welches die oben genannten Konzentrationen an 2-Butenen aufweist. Insbesondere kann als Gemisch I in dem erfindungsgemäßen Verfahren ein Gemisch eingesetzt werden, welches als Fraktion IV in Verfahrensschritt c) von DE 102005062722 erhalten wird.

Bevorzugt eingesetzt werden $C_4$-Fraktionen aus Steamcrackern, die primär zur Produktion von Ethen und Propen betrieben werden und in denen als Rohstoffe beispielsweise Raffineriegase, Naphtha, Gasöl, LPG (liquified petroleum gas) und NGL (natural gas liquid) eingesetzt werden, oder $C_4$-Fraktionen aus Katcrackern. Die als Nebenprodukt anfallenden $C_4$-Schnitte enthalten je nach Crack-Verfahren unterschiedliche Mengen an Isobuten. Weitere Hauptbestandteile sind 1,3-Butadien, 1-Buten, c-2-Buten, t-2-Buten, n-Butan und i-Butan. Typische Isobuten-Gehalte in der $C_4$-Fraktion liegen bei $C_4$-Fraktionen aus Steam-Crackern bei 18 bis 35 Massen-%, bei Fluid-Katcrackern (FCC) bei 10 bis 20 Massen-%.

Für das erfindungsgemäße Verfahren ist es vorteilhaft, mehrfach ungesättigte Kohlenwasserstoffe wie 1,3-Butadien aus dem Einsatzgemisch zu entfernen. Dies kann nach bekannten Verfahren, beispielsweise durch Extraktion, Extraktivdestillation oder Komplexbildung erfolgen (vgl. K.Weissermel, H.J. Arpe, Industrielle Organische Chemie, Wiley-VCH, 5. Auflage, 1998, Seiten 119 bis 121).

Eine Alternative zur Abtrennung der mehrfach ungesättigten Kohlenwasserstoffe ist eine selektive chemische Umsetzung. So kann beispielsweise 1,3-Butadien selektiv zu linearen Butenen hydriert werden, wie z. B. beschrieben in EP 0 523 482. Auch durch selektive Umsetzungen des 1,3-Butadiens, zum Beispiel Dimerisierung zum Cyclooctadien, Trimerisierung zum Cyclododecadien, Polymerisations- oder Telomerisationsreaktionen, kann das 1,3-Butadien zumindest teilweise entfernt werden. Wurde ein Crack-$C_4$-Schnitt als Rohstoff eingesetzt, bleibt in allen Fällen ein Kohlenwasserstoffgemisch (z. B. Raffinat I oder hydriertes Crack-$C_4$ (HCC$_4$)) zurück, das hauptsächlich die gesättigten Kohlenwasserstoffe, n-Butan und Isobutan und die Olefine Isobuten, 1-Buten und 2-Butene enthält.

Bevorzugt werden Gemische I eingesetzt, bei denen in einer zusätzlichen Reinigungsstufe in den $C_4$-Kohlenwasserstoffströmen enthaltene mehrfach ungesättigte Kohlenwasserstoffe katalytisch selektiv hydriert werden. Besonders bevorzugt ist zumindest eine solche Reinigungsstufe vorgesehen wenn nicht ausgeschlossen werden kann, dass die eingesetzten technischen $C_4$-Kohlenwasserstoffströme mehrfach ungesättigte Kohlenwasserstoffe aufweisen.

Bei den mehrfach ungesättigten Kohlenwasserstoffen handelt es sich hauptsächlich um 1,3-Butadien; 1,2-Butadien, Butenin und 1-Butin sind, wenn überhaupt, in deutlich geringerer Menge enthalten. Die Hydrierung kann in einem ein- oder mehrstufigen Hydrierprozess in der Flüssigphase an einem Palladiumkontakt erfolgen. Zu Absenkung des Gehalts an 1,3-Butadien unter vorzugsweise 1000 wppm wird dabei in der letzten Stufe der Hydrierung mit Zusatz eines Moderators gearbeitet, der die Selektivität des Palladiumkontakts erhöht. Bevorzugt wird als Moderator Kohlenmonoxid eingesetzt, das in einem Anteil von 0,05 bis 100 Gew.-ppm (wppm) zugesetzt wird. Der Gehalt an mehrfach ungesättigten Kohlenwasserstoffen sollte im Zulauf zu dieser Stufe unter 1 %, bevorzugt unter 0,5 %, betragen. In der Literatur ist diese Art der Selektivhydrierung von Restgehalten an 1,3-Butadien unter der Bezeichnung SHP (selective hydrogenation process) bekannt (vergleiche EP 0 081 041; Erdöl, Kohle, Erdgas, Petrochem. 1986,39,73).

Sind in den Isobuten-haltigen $C_4$-Strömen Mengen größer 1 Massen-% an mehrfach ungesättigten Kohlenwasserstoffe wie 1,3-Butadien enthalten, werden sie vorzugsweise in vorgeschalteten Hydrierungen umgesetzt. Diese Hydrierungen werden bevorzugt in der Flüssigphase an einem Palladiumkontakt durchgeführt. Je nach Gehalt an ungesättigten Kohlenwasserstoffen kann die Hydrierung in mehreren Stufen durchgeführt werden. Zur Umsetzung von Crack-$C_4$ aus einem Steam-Cracker mit einem Gehalt an 1,3-Butadien von typischerweise 38 bis 45 % hat sich eine zweistufige Ausführung der Hydrierung bewährt. Dabei können einzelne oder alle Stufen mit einer teilweisen Produktrückführung ausgestattet sein. Im Austrag sind so Konzentrationen an 1,3-Butadien kleiner 1 % erhältlich, so dass eine weitere Umsetzung in einer Selektivhydrierung (SHP) erfolgen kann.

Die in dem erfindungsgemäßen Verfahren eingesetzten Gemische I weisen bevorzugt die folgenden Zusammensetzungen auf, wobei je nach Gehalt an mehrfach ungesättigten Kohlenwasserstoffen eine Hydrierung oder Selektivhydrierung durchgeführt wird.

**Tabelle 1:** Typische Zusammensetzungen von technischen Kohlenwasserstoffgemischen, die im erfindungsgemäßen Verfahren eingesetzt werden können.

| | Steamcracker | | Steamcracker | | Katcracker | |
|---|---|---|---|---|---|---|
| Komponente | $HCC_4$ | $HCC_4$ / SHP | Raff. I | Raff. I / SHP | $CC_4$ | $CC_4$ / SHP |
| Isobutan [Massen-%] | 1-4.5 | 1-4.5 | 1.5 - 8 | 1.5 - 8 | 37 | 37 |
| n-Butan [Massen-%] | 5-8 | 5-8 | 6-15 | 6-15 | 13 | 13 |
| trans-Buten [Massen-%] | 18-21 | 18-21 | 7-10 | 7 - 10 | 12 | 12 |
| 1-Buten [Massen-%] | 35-45 | 35-45 | 15 - 35 | 15-35 | 12 | 12 |
| Isobuten [Massen-%] | 22-28 | 22-28 | 33-50 | 33-50 | 15 | 15 |
| cis-Buten [Massen-%] | 5-9 | 5-9 | 4-8 | 4-8 | 11 | 11 |

(fortgesetzt)

| | Steamcracker | | Steamcracker | | Katcracker | |
|---|---|---|---|---|---|---|
| Komponente | HCC$_4$ | HCC$_4$ / SHP | Raff. I | Raff. I / SHP | CC$_4$ | CC$_4$ / SHP |
| 1,3-Butadien [wppm] | 500- 8000 | 0-50 | 50-8000 | 0-50 | < 10000 | 0-50 |

Erläuterung:
- HCC$_4$: typisch für eine C$_4$ Mischung, die aus dem Crack-C$_4$ eines Steamcrackers (High Severity) nach der Hydrierung des 1,3-Butadiens ohne zusätzliche Moderation des Katalysators erhalten wird.
- HCC$_4$ / SHP: Zusammensetzung HCC$_4$, bei dem Reste an 1,3-Butadien in einer SHP weiter reduziert wurden.
- Raff. I (Raffinat I): typisch für eine C$_4$ Mischung, die aus dem Crack-C$_4$ eines Steamcrackers (High Severity) nach der Abtrennung des 1,3-Butadiens, beispielsweise durch eine NMP-Extraktivrektifikation, erhalten wird.
- Raff. I / SHP: Zusammensetzung Raff. I, bei dem Reste an 1,3-Butadien in einer SHP weiter reduziert wurden.
- CC$_4$: typische Zusammensetzung eines Crack-C$_4$, das aus einem Katcracker erhalten wird.
CC$_4$ / SHP: Zusammensetzung CC$_4$, bei dem Reste an 1,3-Butadien in einer SHP weiter reduziert wurden.

Das Raffinat I bzw. HCC$_4$ ist, neben anderen, ein bevorzugt eingesetztes Isobuten-haltiges Kohlenwasserstoffgemisch im Rahmen dieser Erfindung. Da Anlagen zur Aufarbeitung von C$_4$-Kohlenwasserstoffen in der Regel als Strang (Verbund mehrerer Anlagen) aufgebaut sind, ist es jedoch möglich, dass das Raffinat I bzw. HCC$_4$ vor dem Eintritt in das erfindungsgemäße Verfahren eine oder mehrere andere Prozessstufen durchläuft. Diese Prozessstufen können beispielsweise auch Verfahren bzw. Verfahrensschritte sein, wie sie in den Ausführungsformen zum Verfahrensschritt a) beschrieben worden sind. In dem erfindungsgemäßen Verfahren einsetzbare C$_4$-Kohlenwasserstoffgemische können auch solche sein, wie sie aus Verfahren gemäß den Ausführungsformen der Verfahrensschritt a) und anschließender Trennung gemäß Verfahrensschritt b) erhalten werden. Insbesondere können auch solche Gemische eingesetzt werden, wie sie bei der Herstellung von TBA aus Isobuten nach Abtrennung des TBA erhalten werden. Auf diese Weise kann jeweils ein individuell angepasstes Gesamtkonzept zur Aufarbeitung mit dem entsprechenden Produktportfolio realisiert werden.

Typische Verfahren, die den erfindungsgemäßen Verfahren vorgelagert sein können, sind Wasserwäschen, Reinigungsverfahren an Adsorptionsmitteln, Trocknungsverfahren und Destillationen.

## Wasserwäsche

Durch eine Wasserwäsche können hydrophile Komponenten aus dem einzusetzenden technischen Kohlenwasserstoffgemisch, enthaltend Isobuten und lineare Butene, ganz oder teilweise entfernt werden, beispielsweise Stickstoffkomponenten. Beispiele für Stickstoffkomponenten sind Acetonitil oder N-Methylpyrrolidon (die z. B. aus einer 1,3-Butadien-Extraktivdestilltion stammen können). Auch Sauerstoffverbindungen (z. B. Aceton aus einer FCC-Einheit) können zum Teil über eine Wasserwäsche entfernt werden. Der Isobuten-haltige Kohlenwasserstoffstrom ist nach einer Wasserwäsche mit Wasser gesättigt. Um eine Zweiphasigkeit in den nachfolgenden Prozessschritten im Reaktor zu vermeiden, sollte dort die Reaktionstemperatur um ca. 10 °C über der Temperatur der Wasserwäsche liegen.

## Adsorptionsmittel

Adsorptionsmittel werden eingesetzt, um Verunreinigungen zu entfernen. Dies kann beispielsweise vorteilhaft sein, wenn in einem der Prozessschritte Edelmetallkatalysatoren zum Einsatz kommen. Oftmals werden Stickstoff oder Schwefelverbindungen über vorgeschaltete Adsorber entfernt. Beispiele für Adsorptionsmittel sind Aluminiumoxide, Molekularsiebe, Zeolithe, Aktivkohle, mit Metallen imprägnierte Tonerden. Adsorptionsmittel werden von diversen Firmen vertrieben, beispielsweise der Firma Alcoa (Selexsorb®).

## Trocknung

Im Isobuten-haltigen Kohlenwasserstoffgemisch gegebenenfalls enthaltenes Wasser, das beispielsweise aus der Wasserwäsche stammen kann, kann durch bekannte Verfahren zur Trocknung entfernt werden. Geeignete Verfahren sind beispielsweise die destillative Abtrennung des Wassers als Azeotrop. Dabei kann oftmals ein Azeotrop mit enthaltenen C$_4$-Kohlenwasserstoffen ausgenutzt werden oder es können Schleppmittel zugesetzt werden.

Die Trocknung des Kohlenwasserstoffgemisches kann aus diversen Gründen vorteilhaft sein, beispielsweise zur Verringerung der Bildung von Alkoholen (hauptsächlich tert.-Butylalkohol) in Schritt a) bei den Ausführungsformen 2 und 3, zur Verhinderung einer (ungesteuerten) Wassermoderation in der Butenoligomerisierung (Ausführungsform 3 des Schritts a), zur Vermeidung von technischen Problemen durch Abscheidung von Wasser oder zur Vermeidung von Eisbildung bei niedrigen Temperaturen (z. B. Zwischenlagerung).

## Destillation

Destillationsschritte können beispielsweise genutzt werden, um Verunreinigungen abzutrennen (beispielsweise Leichtsieder wie $C_3$-Kohlenwasserstoffe, Schwersieder wie $C_5$-Kohlenwasserstoffe) oder um Fraktionen mit unterschiedlichen Isobutenkonzentrationen zu erhalten. Dies kann sowohl direkt mit dem Raffinat I bzw. dem $HCC_4$ erfolgen oder nachdem eine oder mehrere andere Prozessstufen durchlaufen wurden. Durch direkte Destillation des Raffinats I bzw. des $HCC_4$s ist beispielsweise eine Trennung in eine an 2-Butenen und n-Butan verarmte, Isobuten reichere Fraktion möglich.

Je nach Zusammensetzung des einzusetzenden technischen Kohlenwasserstoffgemisches und/oder nach den Reinheiten der Zielprodukte kann das technische Kohlenwasserstoffgemisch also direkt in den Schritt a) des erfindungsgemäßen Verfahrens oder aber erst nach einer Vorbehandlung durch eines oder mehrere der vorgenannten Verfahren eingesetzt werden.

Das erfindungsgemäße Verfahren kann eigenständig betrieben werden. Insbesondere aber kann das erfindungsgemäße Verfahren als Verfahrensstufe d) in DE 10 2005 062722 oder als Verfahrensstufe d) in DE 10 2005 062700 eingesetzt werden, wobei als Gemisch I des erfindungsgemäßen Verfahrens die Fraktion IV der genannten Schriften eingesetzt wird.

## Beschreibung der Figuren

An Hand der Figuren Fig. 1 bis 4 wird das erfindungsgemäße Verfahren nachfolgend näher erläutert, ohne dass das Verfahren auf die dort beispielhaft abgebildeten Ausführungsarten beschränkt sein soll.

In Fig. 1 ist der Verlauf von Isobuten- und 2-Buten-Konzentration bei einem Versuch zur ETBE-Synthese bei 42°C und einer Ausgangskonzentration von 7500 wppm Isobuten in einem äquimolaren Gemisch aus 1-Buten und Isobutan, abgebildet. Es ist zu erkennen, dass mit längerer Verweilzeit die Konzentration an Isobuten ab und an 2-Buten zunimmt.

In Fig. 2 sind graphisch die in Beispiel 1 ermittelten Datensätze dargestellt. Die gestrichelten Linien grenzen einen Bereich ab, in dem das erhaltene 1-Buten eine Konzentration an Isobuten und 2-Butenen aufweist, die der gewünschten Konzentration entspricht (kleiner 2000 ppm Isobuten und kleiner 500 ppm 2-Butene [jeweils Massen-ppm]).

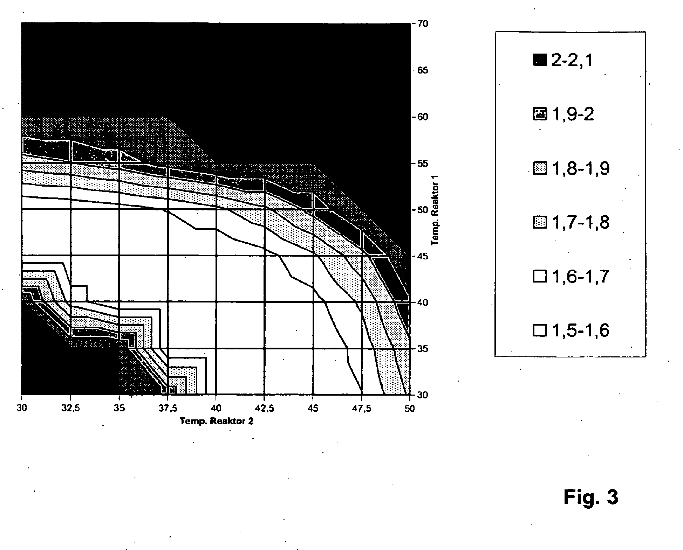

Fig. 3 zeigt eine graphische Darstellung der Zielfunktion für eine vorgegebene Menge von zuzugebendem Alkohol. Dabei ist in der Darstellung die Temperatur im ersten Reaktor gegen die Temperatur im zweiten Reaktor aufgetragen. Aus den mit verschiedenen Mustern gekennzeichneten Bereichen lassen sich Temperaturpaare für den Betrieb der Reaktoren 1 und 2 entnehmen, mit welchem spezifikationsgerechten Reaktionsprodukt II erhalten werden kann. Wertepaare die im einheitlich schwarzen Bereich liegen, führen zu nicht spezifikationsgerechtem Reaktionsprodukt II.

In Fig. 4 ist schematisch eine Ausführungsform des erfindungsgemäßen Verfahrens dargestellt. Das Gemisch I wird in den ersten Reaktor R1 einer Kaskade von zwei Reaktoren eingespeist, in den auch Ethanol eingespeist wird. Der Reaktoraustrag aus dem ersten Reaktor wird in den zweiten Reaktor R2 gefahren. Der Austrag aus dem zweiten Reaktor II wird in eine Destillationskolonne K1 überführt, die mit einem Kondensator W-b2 für das Kopfprodukt und einem Sumpfverdampfer W-b1 ausgestattet ist. Ein Teil des Kopfproduktes wird als Rücklauf in die Kolonne zurückgefahren. Als Sumpfprodukt wird das Produkt III, hauptsächlich ETBE und gegebenenfalls Restmengen an Ethanol erhalten. Als Kopfprodukt wird der Strom IV abgenommen, der 1-Buten und gegebenenfalls noch Ethanol aufweist. Enthält der Strom Ethanol kann dieser Strom unten in eine Extraktionskolonne E1 gefahren werden, in die im Gegenstrom ein Extraktionsmittel, wie z. B. Wasser, über den am Kopf befindlichen Zulauf E-f1 eingespeist wird, welches über den Ablauf E-f2 am Sumpf der Kolonne entnommen wird. Am Kopf der Kolonne wird als Produkt der Extraktion der Strom V erhalten, der 1-Buten aufweist und der einer weiteren Aufarbeitung zugeführt werden kann..

Die folgenden Beispiele sollen die Erfindung erläutern, ohne den Schutzbereich der sich aus den Patentansprüchen und der Beschreibung ergibt, einzuschränken.

## Beispiel 1: Erstellen von Datensätzen und Auswahl von geeigneten Datensätzen

Mit der Simulationssoftware Aspen Plus (12.1, Aspen Technology Inc.) wurden einige Datensätze unter Verwendung der oben aufgeführten Gleichungen erstellt. Es wurden wiederum die nachfolgend aufgeführten Reaktionen bzw. Nebenreaktionen zu Grunde gelegt:

$$\text{1-Buten-Isomerisierung:} \quad \text{1-Buten} \; \underset{r6'}{\overset{r6}{\rightleftharpoons}} \; \text{2-Buten}$$

$$\text{ETBE-Synthese:} \quad \text{Isobuten + Ethanol} \; \underset{r1'}{\overset{r1}{\rightleftharpoons}} \; \text{ETBE}$$

Diese Reaktionen wurden kinetisch untersucht. Aus der Untersuchung wurden Geschwindigkeitsansätze mit einer guten ($R^2 > 0{,}98$) Übereinstimmung mit den gemessenen Werten erhalten. Die Aktivitäten wurden unter Verwendung des Aktivitätskoeffizientenmodells NRTL-RK berechnet. Die Geschwindigkeitskonstanten folgen dem Arrhenius-Gesetz:

$$k_i = k_{0,i} \cdot \exp\left(\frac{-E_{A,i}}{R \cdot T}\right)$$

Die erhaltenen Kinetiken wurden für die Berechnung der Konzentrationen am Ausgang des zweiten Reaktors ausgehend von den Eingangskonzentrationen und den Bedingungen in den technischen Reaktoren eingesetzt.
Dabei wurden folgende Betriebsparameter festgelegt:

Katalysatorvolumen Reaktor 1: 1,8
Katalysatorvolumen Reaktor 2: 2,8
Molares Verhältnis von Isobuten zu 1-Buten: 1 : 9
Zulauf zum Reaktor 1: 2,3 kg/h
Zulauf Ethanol: 250 g/h

Variiert wurden die folgenden Werte in den angegebenen Bereichen:

Isobutenkonzentration: 0,5 bis 1,5 Massen-%
Eingangstemperatur Reaktor 1: 30 bis 60 °C
Eingangstemperatur Reaktor 2: 30 bis 60 °C

Aus dieser Parametervariation wurden 10000 Datensätze berechnet. Aus den entstandenen Wertepaaren (Isobuten, 2-Buten in 1-Buten) wurde die Funktion F(x) berechnet.
Die zwei technischen Reaktoren wurden mit der bekannten Differentialgleichung für die Bilanz eines polytropen Rohrreaktors berechnet. Für die Wärmebilanz wurden die Enthalpien der jeweiligen Reaktionen verwendet.
Fig. 2 zeigt graphisch die aus den Datensätzen abgeleiteten Funktionen, aus denen leicht diejenigen Werte aufgewählt werden können, die zu einem Reaktionsprodukt II führen, welche innerhalb der gewünschten Konzentrationen an Isobuten und 2-Butenen liegen. Auf diese einfache Weise ist es beispielsweise möglich die optimalen Temperaturbedingungen für die Reaktoren in Abhängigkeit von der Anfangskonzentration an Isobuten zu ermitteln.
Für eine Anfangskonzentration an Isobuten von 0,55 Massen-% wird durch Anwendung der Zielfunktion für den Datensatz, der eine Temperatur von 40 °C sowohl im ersten als auch im zweiten Reaktor aufweist, ein minimaler Wert für die Zielfunktion von 1,39 erhalten, der deutlich unterhalb des Grenzwertes von 2,07 liegt. Als beste Temperaturführung für diesen Fall wird deshalb eine Eingangstemperatur von 40 °C sowohl im ersten als auch im zweiten Reaktor erhalten. Fig. 3 zeigt eine graphische Darstellung der Zielfunktion für die in Beispiel 1 gewählten Parameter bei einer Anfangskonzentration an Isobuten von 0,55 Massen-%.

**Patentansprüche**

1. Verfahren zur Herstellung von Alkyl-tert.-butylether aus einem Gemisch I, welches Isobuten und 1-Buten aufweist, durch sauer katalysierte Umsetzung des Isobutens mit Alkohol in zumindest zwei hintereinander geschalteten Reaktoren unter Erhalt eines Reaktionsproduktes II, welches gewünschte Konzentrationen an 2-Butenen und Isobuten in Bezug auf das enthaltene 1-Buten nicht überschreitet,
**dadurch gekennzeichnet,**

**dass** die Umsetzung bei Temperaturen in °C am Eingang der Reaktoren und unter Zugabe einer Alkoholmenge zur Umsetzung durchgeführt wird, die jeweils um maximal 10 % von den Werten abweichen, die erhältlich sind durch die Schritte:

a) Ermitteln von mehreren Datensätzen bei denen Wertepaare für die Konzentrationen an 2-Butenen und Isobuten in Reaktionsprodukt II bestimmten Werten für die zugeführte Masse an Gemisch I zur Umsetzung, für die Isobuten-Konzentration in Gemisch I, für die Konzentration von 1-Buten im Gemischs I, für die der Umsetzung zugeführte Alkoholmenge und für die Katalysatorvolumina in den Reaktoren zugeordnet sind, durch entsprechend häufiges Durchführen einer Umsetzung eines Gemischs I, wobei bei den Durchführungen die Konzentration von 1-Buten, die Isobuten-Konzentration, das Katalysatorvolumina, die zugeführte Masse an Gemisch I zur Umsetzung, die Temperaturen am Eingang der Reaktoren und/oder die der Umsetzung zugeführten Menge an Alkohol variiert wird,

b) Auswahl eines Datensatzes, der Werte aufweist, die den tatsächlichen Bedingungen bei der Umsetzung in Bezug auf die zugeführte Masse an Gemisch I zur Umsetzung, die Isobuten-Konzentration in Gemisch I, die Konzentration von 1-Buten in Gemisch I und die Katalysatorvolumina mit einer Abweichung von maximal 5 % entsprechen und dem ein Wertepaar für die Konzentrationen an Isobuten und 2-Butenen im Reaktionsprodukt II zugeordnet ist, welche die gewünschten Konzentrationen an 2-Butenen und Isobuten in Bezug auf das enthaltene 1-Buten nicht überschreiten, wobei in dem Datensatz Werte für die Temperaturen am Eingang der Reaktoren sowie für die der Umsetzung zuzuführenden Menge an Alkohol enthalten sind.

2. Verfahren nach Anspruch 1,
   **dadurch gekennzeichnet,**
   **dass** die gewünschten Konzentrationen an Isobuten, jeweils bezogen auf das enthaltene 1-Buten, kleiner 5000 wppm und an 2-Butenen kleiner 1000 wppm betragen.

3. Verfahren nach Anspruch 2,
   **dadurch gekennzeichnet,**
   **dass** die gewünschte Konzentrationen an Isobuten, jeweils bezogen auf das enthaltene 1-Buten, kleiner 2000 wppm und an 2-Butenen kleiner 500 wppm betragen.

4. Verfahren nach zumindest einem der Ansprüche 1 bis 3,
   **dadurch gekennzeichnet,**
   **dass** die Umsetzung in Schritt a) als Simulation durchgeführt wird.

5. Verfahren nach zumindest einem der Ansprüche 1 bis 3,
   **dadurch gekennzeichnet,**
   **dass** in Schritt a) zumindest 6000 Datensätze ermittelt werden.

6. Verfahren nach zumindest einem der Ansprüche 1 bis 4,
   **dadurch gekennzeichnet,**
   **dass** in Schritt a) bei der Ermittlung der Datensätze die Konzentration von 1-Buten und das Katalysatorvolumen konstant gehalten wird.

7. Verfahren nach zumindest einem der Ansprüche 1 bis 5,
   **dadurch gekennzeichnet,**
   **dass** die Auswahl in Schritt b) unter Verwendung einer Zielfunktion erfolgt.

8. Verfahren nach zumindest einem der Ansprüche 1 bis 6,
   **dadurch gekennzeichnet,**
   **dass** als Alkohol Methanol oder Ethanol eingesetzt wird.

9. Verfahren nach zumindest einem der Ansprüche 1 bis 7,
   **dadurch gekennzeichnet,**
   **dass** als Katalysator ein Ionenaustauscherharz eingesetzt wird.

10. Verfahren nach zumindest einem der Ansprüche 1 bis 8,
    **dadurch gekennzeichnet,**
    **dass** das erhaltene Reaktionsprodukt destillativ in einen Alkyl-tert.-butylether III und einen 1-Buten aufweisenden Strom IV getrennt wird.

11. Verfahren nach Anspruch 9,
**dadurch gekennzeichnet,**
**dass** ein 1-Buten aufweisender Strom IV, der weniger als 2000 wppm Isobuten und weniger als 500 wppm 2-Buten, jeweils bezogen auf das enthaltene 1-Buten, aufweist, hergestellt wird.

**Claims**

1. Process for the preparation of alkyl tert-butyl ether from a mixture I exhibiting isobutene and 1-butene by acid catalysed conversion of the isobutene with alcohol in at least two reactors placed one behind the other with a reaction product II being obtained which does not exceed desired concentrations of 2-butenes and isobutene, based on the 1-butene present,
**characterized in that**
the conversion is carried out at temperatures in °C at the inlet of the reactors and with addition of an amount of alcohol for the conversion which in each case differ by about at most 10% from the values which can be obtained by the steps:

   a) determining various data records in which pairs of values for the concentrations of 2-butenes and isobutene in reaction product II are allocated to specific values for the weight of mixture I fed to the conversion, for the isobutene concentration in mixture I, for the concentration of 1-butene in mixture I, for the amount of alcohol fed to the conversion and for the catalyst volumes in the reactors by carrying out, appropriately frequently, a conversion of a mixture I during which the concentration of 1-butene, the isobutene concentration, the catalyst volumes, the weight of mixture I fed to the conversion, the temperatures at the inlet of the reactors and/or the amount of alcohol fed to the conversion are varied,
   b) selecting a data record which exhibits values which correspond to the actual conditions in the conversion with regard to the weight of mixture I fed to the conversion, the isobutene concentration in mixture I, the concentration of 1-butene in mixture I and the catalyst volumes with a deviation of at most 5% and to which a pair of values for the concentrations of isobutene and 2-butenes in the reaction product II is allocated which do not exceed the desired concentrations of 2-butenes and isobutene, based on the 1-butene present, values for the temperatures at the inlet of the reactors and for the amount of alcohol to be fed to the conversion being present in the data record.

2. Process according to Claim 1,
**characterized in that**
the desired concentrations of isobutene, in each case based on the 1-butene present, are less than 5000 wppm and of 2-butenes are less than 1000 wppm.

3. Process according to Claim 2,
**characterized in that**
the desired concentrations of isobutene, in each case based on the 1-butene present, are less than 2000 wppm and of 2-butenes are less than 500 wppm.

4. Process according to at least one of Claims 1 to 3,
**characterized in that**
the conversion in step a) is carried out as simulation.

5. Process according to at least one of Claims 1 to 3,
**characterized in that**
at least 6000 data records are determined in step a).

6. Process according to at least one of Claims 1 to 4,
**characterized in that**
the concentration of 1-butene and the catalyst volume are kept constant in step a) in the determination of the data records.

7. Process according to at least one of Claims 1 to 5,

**characterized in that**
the selection in step b) is carried out using a target function.

8. Process according to at least one of Claims 1 to 6,
**characterized in that**
methanol or ethanol is used as alcohol.

9. Process according to at least one of Claims 1 to 7,
**characterized in that**
an ion-exchange resin is used as catalyst.

10. Process according to at least one of Claims 1 to 8,
**characterized in that**
the reaction product obtained is separated by distillation into an alkyl tert-butyl ether III and a stream IV exhibiting 1-butene.

11. Process according to Claim 9,
**characterized in that**
a stream IV exhibiting 1-butene is prepared which exhibits less than 2000 wppm of isobutene and less than 500 wppm of 2-butenes, in each case based on the 1-butene present.

**Revendications**

1. Procédé de fabrication d'un éther de tert.-butyle et d'alkyle à partir d'un mélange I, qui comporte de l'isobutène et du 1-butène, par réaction catalysée par un acide de l'isobutène avec un alcool dans au moins deux réacteurs connectés en série, pour obtenir un produit de réaction II, qui ne dépasse pas des concentrations souhaitées en 2-butènes et en isobutène par rapport au 1-butène contenu,
**caractérisé en ce que** la réaction est réalisée à des températures en °C à l'entrée des réacteurs et en ajoutant une quantité d'alcool pour la réaction qui diffèrent au plus de 10 % des valeurs pouvant être obtenues par les étampes :

a) calcul de plusieurs ensembles de données selon lesquels des paires de valeurs pour les concentrations en 2-butènes et en isobutène dans le produit de réaction II sont attribuées à des valeurs déterminées pour la masse introduite de mélange I pour la réaction, pour la concentration en isobutène dans le mélange I, pour la concentration en 1-butène dans le mélange I, pour la quantité d'alcool introduite dans la réaction et pour les volumes de catalyseur dans les réacteurs, par réalisation à une fréquence appropriée d'une réaction d'un mélange I, la concentration en 1-butène, la concentration en isobutène, les volumes de catalyseur, la masse introduite de mélange I pour la réaction, les températures à l'entrée des réacteurs et/ou la quantité d'alcool introduite dans la réaction variant lors des réalisations,
b) choix d'un ensemble de données, qui comporte des valeurs qui correspondent aux conditions réelles lors de la réaction au regard de la masse introduite de mélange I pour la réaction, la concentration en isobutène dans le mélange I, la concentration en 1-butène dans le mélange I et les volumes de catalyseur avec une déviation maximale de 5 %, et auquel est attribuée une paire de valeurs pour les concentrations en isobutène et en 2-butènes dans le produit de réaction II, qui ne dépassent pas les concentrations souhaitées en 2-butènes et en isobutène par rapport au 1-butène contenu, des valeurs pour les températures à l'entrée des réacteurs et pour la quantité d'alcool à introduire dans la réaction étant contenues dans l'ensemble de données.

2. Procédé selon la revendication 1, **caractérisé en ce que** les concentrations souhaitées en isobutène, à chaque fois par rapport au 1-butène contenu, sont inférieures à 5 000 ppm en poids, et en 2-butènes inférieures à 1 000 ppm en poids.

3. Procédé selon la revendication 2, **caractérisé en ce que** les concentrations souhaitées en isobutène, à chaque fois par rapport au 1-butène contenu, sont inférieures à 2 000 ppm en poids, et en 2-butènes inférieures à 500 ppm en poids.

4. Procédé selon au moins l'une quelconque des revendications 1 à 3, **caractérisé en ce que** la réaction à l'étape a) est réalisée sous la forme d'une simulation.

**5.** Procédé selon au moins l'une quelconque des revendications 1 à 3, **caractérisé en ce qu'**au moins 6 000 ensembles de données sont calculés à l'étape a).

**6.** Procédé selon au moins l'une quelconque des revendications 1 à 4, **caractérisé en ce que** la concentration en 1-butène et le volume de catalyseur sont maintenus constants pendant le calcul des ensembles de données à l'étape a).

**7.** Procédé selon au moins 1"une quelconque des revendications 1 à 5, **caractérisé en ce que** le choix à l'étape b) a lieu en utilisant une fonction objectif.

**8.** Procédé selon au moins l'une quelconque des revendications 1 à 6, **caractérisé en ce que** le méthanol ou l'éthanol est utilisé en tant qu'alcool.

**9.** Procédé selon au moins l'une quelconque des revendications 1 à 7, **caractérisé en ce qu'**une résine échangeuse d'ions est utilisée en tant que catalyseur.

**10.** Procédé selon au moins l'une quelconque des revendications 1 à 8, **caractérisé en ce que** le produit de réaction obtenu est séparé par distillation en un éther de tert.-butyle et d'alkyle III et en un courant IV comprenant du 1-butène.

**11.** Procédé selon la revendication 9, **caractérisé en ce qu'**un courant IV comprenant du 1-butène, qui comprend moins de 2 000 ppm en poids d'isobutène et moins de 500 ppm en poids de 2-butènes, à chaque fois par rapport au 1-butène contenu, est fabriqué.

**Fig. 1**

**Fig. 2**

**Fig. 3**

Fig.4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2521964
- US 4161496 A
- EP 729085 A
- DE 102005062722

- DE 10020943
- EP 0523482 A
- EP 0081041 A
- DE 102005062700

**In der Beschreibung aufgeführte Nicht-Patentliteratur**

- **K. WEISSERMEL ; H. J. ARPE.** Industrielle Organische Chemie. Wiley-VCH, 1998, 23-2465-99122-124

- **K.WEISSERMEL ; H.J. ARPE.** Industrielle Organische Chemie. Wiley-VCH, 1998, 119-121